(19) Europäisches Patentamt
European Patent Office
Office européen des brevets

(11) **EP 1 761 804 B1**

(12) **FASCICULE DE BREVET EUROPEEN**

(45) Date de publication et mention
de la délivrance du brevet:
**01.10.2008 Bulletin 2008/40**

(21) Numéro de dépôt: **05778190.8**

(22) Date de dépôt: **27.06.2005**

(51) Int Cl.:
***G01T 1/29*** *(2006.01)*

(86) Numéro de dépôt international:
**PCT/FR2005/050499**

(87) Numéro de publication internationale:
**WO 2006/008409 (26.01.2006 Gazette 2006/04)**

(54) **TOMOGRAPHE A DETECTEURS DE FORME SPECIFIQUE**

TOMOGRAF MIT SPEZIFISCH GEFORMTEN DETEKTOREN

TOMOGRAPH COMPRISING DETECTORS HAVING A SPECIFIC SHAPE

(84) Etats contractants désignés:
**AT BE BG CH CY CZ DE DK EE ES FI FR GB GR HU IE IS IT LI LT LU MC NL PL PT RO SE SI SK TR**

(30) Priorité: **28.06.2004 FR 0451350**

(43) Date de publication de la demande:
**14.03.2007 Bulletin 2007/11**

(73) Titulaire: **Commissariat à l'Energie Atomique 75015 Paris (FR)**

(72) Inventeurs:
• **DREZET, Arnaud**
 **F-25000 Besancon (FR)**

• **MONNET, Olivier**
 **F-38210 TULLINS (FR)**
• **MONTEMONT, Guillaume**
 **F-38000 GRENOBLE (FR)**
• **VERGER, Loïck**
 **F-38000 GRENOBLE (FR)**

(74) Mandataire: **Poulin, Gérard et al Brevatome 3, rue du Docteur Lancereaux 75008 Paris (FR)**

(56) Documents cités:
 **EP-A- 1 408 347** **EP-A- 1 413 898**
 **US-B1- 6 285 028**

EP 1 761 804 B1

## EP 1 761 804 B1

**Description**

## DOMAINE TECHNIQUE ET ART ANTÉRIEUR

L'invention se rapporte au domaine de l'imagerie médicale par tomographie à émissions de positons (TEP), comme décrit dans le document EP 1 408 347 A1, et plus spécifiquement au développement d'un système d'imagerie TEP dédié au petit animal (principalement les rongeurs) permettant d'obtenir une amélioration de la résolution spatiale.

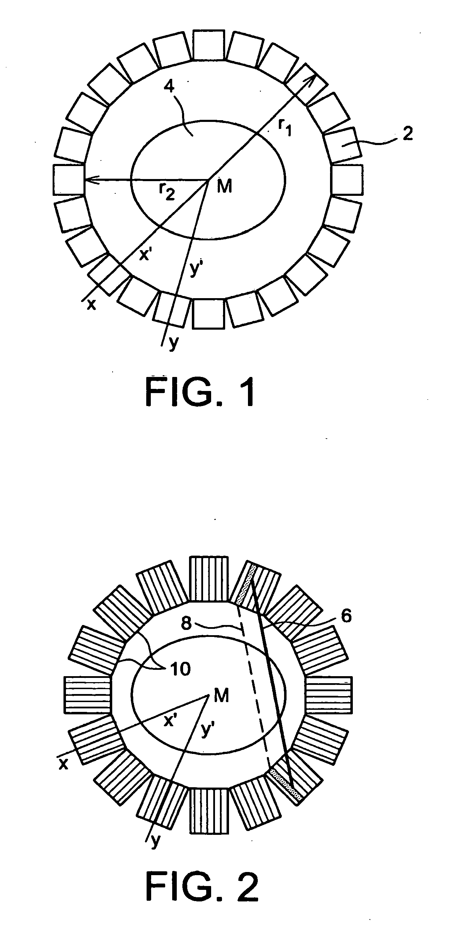

La grande majorité des systèmes TEP utilisent des détecteurs scintillateurs 2 de forme parallélépipédique rectangulaire, assemblés en couronnes, comme illustré sur la figure 1, autour d'un objet à imager 4.

L'objet à imager 4 se comporte en fait comme une source de rayonnements gamma, issus de la désintégration d'un radiotraceur préalablement injecté dans l'objet. Ces rayons $\gamma$ sont émis par paire; à 180° l'un de l'autre, et sont donc détectés par deux détecteurs en coïncidence.

Développés pour les études concernant le corps humain, ces systèmes se révèlent inadaptés pour l'imagerie des rongeurs. En effet, afin de s'adapter aux dimensions anatomiques du petit animal, le diamètre intérieur r2 de la couronne de détection est fortement réduit. Cette réduction du diamètre entraîne une dégradation importante de la résolution spatiale : Il apparaît une dégradation de la localisation due à la pénétration des rayonnements par la tranche des détecteur, baptisée erreur de parallaxe, comme illustré sur la figure 2.

Sur cette figure, les références 6, 8 désignent respectivement une trajectoire réelle et une trajectoire reconstituée, l'écart entre elles constituant l'erreur dite de parallaxe.

Ce phénomène ne devient véritablement gênant que dans les cas où l'objet à imager possède un diamètre proche du système d'imagerie, ce qui est le cas pour les rongeurs. On doit alors utiliser les faces avant 10 des détecteurs (figure 2) comme point de départ de reconstitution des trajectoires des rayons gamma incidents.

Pour contourner cette difficulté, certains ont développé dés détecteurs formés de plusieurs cristaux différents (voir l'article de W.H. Wong, « Designing a stratified detection system for PET caméras », IEEE Transaction on Nuclear science, 1986, 33(1) : p.591-596). Le signal généré est différent suivant le matériau dans lequel l'interaction se produit. Cela permet de localiser le lieu d'interaction.

Ces systèmes, baptisés « phoswich », sont néanmoins complexes à mettre en oeuvre électroniquement pour un gain modéré en résolution spatiale.

L'utilisation d'un matériau semi-conducteur permet d'éviter cette difficulté. En effet, dans un tel détecteur, le signal généré est un ensemble de charges électriques qui sont guidées dans le détecteur par le champ électrique qui y est appliqué. Leur migration dans le détecteur sous l'influence du champ électrique provoque une induction de charges aux électrodes du détecteur. Il s'agit de localiser lé plus précisément possible la zone d'induction sur ces électrodes. Pour cela, on peut agir géométriquement sur la forme des électrodes en les segmentant.

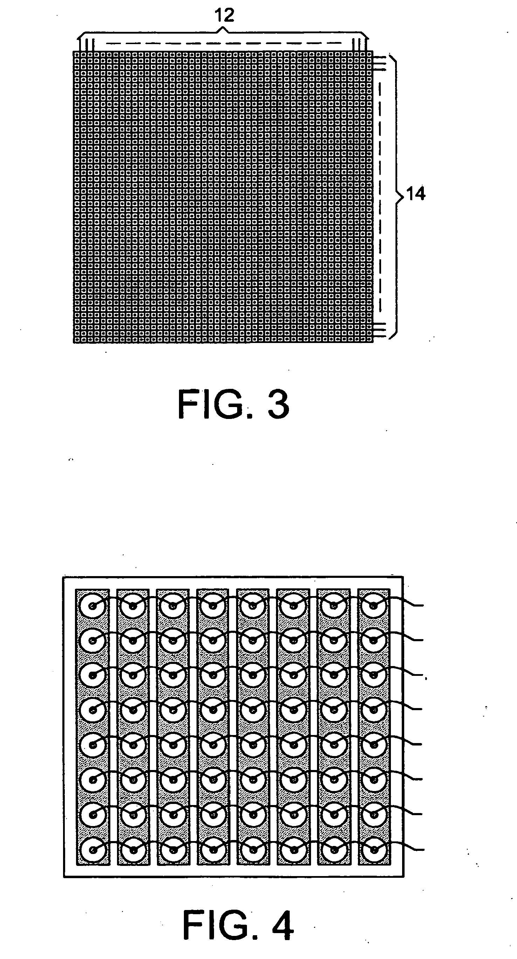

La structuré d'électrodes segmentées la plus répandue consiste en une anode (ou cathode) en damier, de côté n, soit $n^2$ segments (ou « strips ») comme illustré sur la figure 3, et d'une cathode (ou anode) pleine face. Ces $n^2$ segments sont reliés à autant de voies 12, 14 de traitement électronique. Pour une segmentation fine, comme celle requise pour imager un petit animal, cela entraîne une densité électronique très importante. Dans la pratiqué, cette structure présente un coût et une complexité de gestion des voies électroniques qui sont prohibitifs pour l'imagerie TEP des petits animaux.

Pour simplifier le traitement de l'information, il éxiste des structures permettant de réduire ces $n^2$ voies électroniques sur l'anode (ou cathode) à 2n voies, comme illustré sur la figure 4. L'électronique est allégée, mais l'implantation de la connectique est délicate à réaliser, dans la mesure où le système final est constitué de plusieurs détecteurs empilés les uns sur les autres.

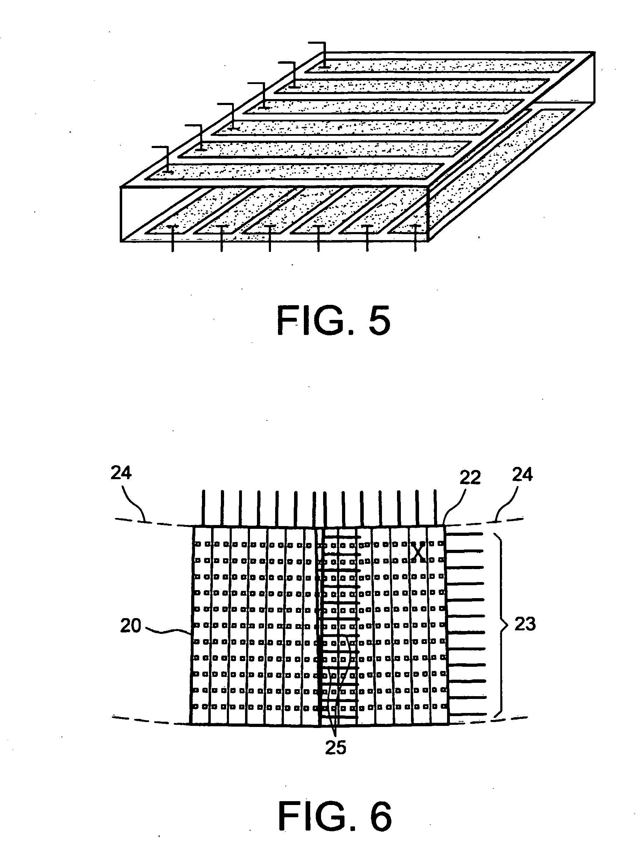

Parmi les structures existantes, une solution à ce problème met en oeuvre la segmentation à la fois de l'anode et de la cathode, comme illustré sur la figure 5.

Les segments anodiques et cathodiques, disposés perpendiculairement, sont toujours au nombre de 2n. Dans le cadre de l'application TEP au petit animal, cet agencement souffre d'un double inconvénient.

Dans un système TEP complet, plusieurs structures de base 20, 22 (figure 6) portant les électrodes segmentées doivent être disposées les unes à côté des autres pour former la couronne de détection. Avec une structure d'électrodes croisées perpendiculairement, la connectique 23 d'une famille d'électrodes (anode ou cathode) se fait nécessairement à l'intérieur dé la couronne. Cela représente une difficulté importante pour le montage, ainsi que de l'espace de détection perdu.

Le schéma de la figure 6 illustré ce problème. Il représenté deux détecteurs 20, 22 consécutifs, « strippés », constitutifs d'une couronne 24 de détection complète. On remarque le problème d'encombrement existant, en particulier, entré les voies électroniques horizontales 25 sortant du détecteur 20 et le détecteur 22, le détecteur 20 étant à son tour gêné par les connexions du détecteur, non représenté sur la figure, situé sur son autre côté.

Par ailleurs, dans un tel système, le nombre total de voies demeure élevé, malgré le gain effectué en passant de $n^2$ voies à 2n voies.

Il se pose donc le problème d'éncombrement de la connéctique, et un problème dû au nombre de voies.

## EXPOSÉ DE L'INVENTION

Afin de résoudre le problème de la dégradation de la résolution spatiale, tout en dépassant les limites rencontrées par les systèmes développés jusque-là, la présente invention propose un agencement géométrique spécifique des électrodes.

L'invention permet d'obtenir une très bonne résolution spatiale pour un coût électronique minimisé.

Pour éviter que des fils de connexion ne soient présents dans la zone de détection, l'invention propose de présenter l'ensemble des sorties anodiques et cathodiques sur le pourtour extérieur de la couronne de détecteurs.

L'invention concerne donc un dispositif détecteur de type tomographe, comportant une pluralité d'éléments détecteurs, disposés en forme de couronne, des électrodes sur une face avant et une face arrière, formant anode et cathode de ces éléments, chaque électrode étant munie de moyens de connexion, l'ensemble de ces moyens de connexion étant localisés sur le pourtour extérieur de ladite couronne d'éléments détecteurs.

Les électrodes peuvent avoir la forme de segments, l'ensemble des segments recouvrant une face d'un élément détecteur étant disposé selon une direction inclinée par rapport à l'ensemble des segments recouvrant l'autre face dudit élément détecteur.

La couronne peut avoir un pourtour intérieur sensiblement en forme de cercle, un pourtour extérieur sensiblement en forme de cercle, ces deux cercles étant concentriques, les segments étant inclinés, par rapport à un rayon du pourtour extérieur, d'un angle inférieur à celui délimité par ledit rayon et une tangente (T1, T2) au cercle intérieur qui rejoint ledit rayon au point d'intersection de ce rayon sur le cercle extérieur.

L'invention concerne également un dispositif détecteur de type tomographe, caractérisé en ce qu'il comporte :

- une pluralité d'éléments détecteurs, disposés en forme de couronne, cette couronne ayant un pourtour intérieur en forme de cercle, un pourtour extérieur en forme de cercle, ces deux cercles étant concentriques,
- des électrodes sur une face avant et une face arrière, formant anode et cathode de ces éléments détecteurs, les électrodes d'une face étant inclinées, par rapport à un rayon du pourtour extérieur, d'un angle inférieur à celui délimité par ledit rayon et une tangente au cercle intérieur qui rejoint ledit rayon au point d'intersection de ce rayon sur le cercle extérieur.

Chaque électrode peut en outre être munie de moyens de connexion, l'ensemble de ces moyens de connexion étant localisés sur le pourtour extérieur de ladite couronne d'éléments détecteurs.

Selon l'invention, on propose donc de disposer les électrodes, de façon à ramener toute la connectique sur un seul côté du détecteur, le côté extérieur d'une couronne de détection.

Dans un dispositif selon l'invention, au moins une partie des segments peut recouvrir au moins deux détecteurs ou chaque électrode peut être commune à au moins deux éléments détecteurs, par exemple entre un et 20 détecteurs.

Certaines bandes ou certains segments peuvent donc être prolongées sur les détecteurs voisins : le nombre de connexions par détecteur est ainsi réduit.

Les électrodes sont par exemple inclinées, sur chaque face, d'un angle inférieur à $\beta 1$ ou $\beta 2$, par rapport à un axe radial passant par l'axe central du système et l'arête de chaque détecteur située dans un plan perpendiculaire à cet axe central, $\beta 1$ et $\beta 2$ étant chacun respectivement défini comme l'angle entre un rayon extérieur du dispositif et une tangente à la circonférence intérieure du dispositif.

Par exemple $\beta 1 = \beta 2 \approx 52,5°$.

Les électrodes des faces avant et arrière des éléments détecteurs peuvent être disposées en forme d'éventails, définissant deux éventails croisés.

Les éléments détecteurs sont de préférence en matériau semi-conducteur, par exemple en matériau CdZnTe, ou CdTe, ou GaAs, ou Ge, ou HgI2, ou Si.

La couronne de détecteurs a par exemple un rayon intérieur compris entre 15 mm et 100 mm et un rayon extérieur compris entre 35 mm et 400 mm.

L'invention permet de réaliser un dispositif de type tomographe à connexions simplifiées. Un tel dispositif est notamment bien adapté au travail sur de petits.animaux tels que des rongeurs.

## BREVE DESCRIPTION DES DESSINS

Les figures 1-6 décrivent des dispositifs de l'art antérieur.

Les figures 7A, 7B, 8 décrivent un dispositif selon l'invention.

## EXPOSÉ DÉTAILLÉ DE MODES DE RÉALISATION PARTICULIERS

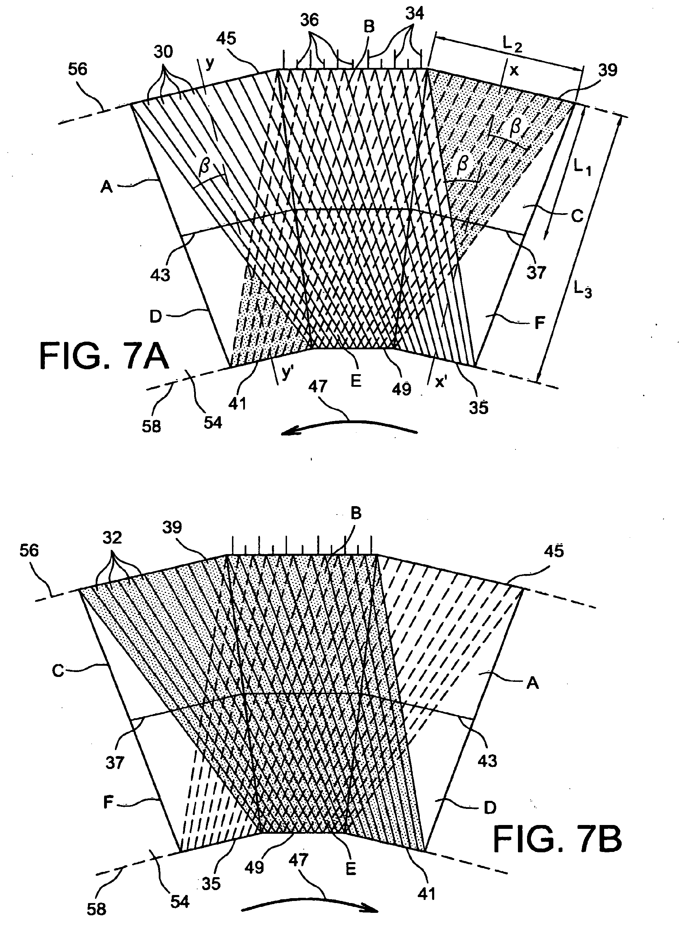

Un exemple d'un dispositif de détection selon l'invention est illustré sur les figures 7A et 7B, la figure 7A représentant la face avant d'un ensemble de blocs détecteurs A - F (par exemple côté cathode) et la figure 7B la face arrière de ce même ensemble (par exemple côté anode).

Les éléments de détection A, B, C, D, E, F, sont représentés par des trapèzes. Sur ces figures 7A et 7B, ces éléments de détection forment une portion angulaire d'une couronne 54 de détection représentée en traits interrompus 56, 58 figurant respectivement le côté extérieur et le côté intérieur de la couronne. Chacun des pourtours intérieur et extérieur est de forme sensiblement circulaire. Cette couronne 54 se prolonge latéralement par encore d'autres éléments détecteurs, non représentés.

Les références 30 et 32 désignent les électrodes qui interviennent dans la détection des interactions ayant lieu dans ces éléments détecteurs.

Globalement, ces électrodes 30, 32 sont segmentées en forme d'éventails croisés, les électrodes situées sur une face des détecteurs étant orientées selon une direction, par rapport à un parcours de la couronne de détecteurs dans un certain sens (sens contraire des aiguilles d'une monstre, repéré par la flèche 47 sur la figure 7A), et les électrodes situées sur l'autre face étant orientées dans l'autre sens par rapport à ce même parcours.

Les électrodes sont donc inclinées d'un angle d'inclinaison $\beta$, ou $=\beta$, par rapport à un axe XX' ou YY' radial qui passe par l'axe central du système et, sensiblement, par le milieu de l'arête 35, 37, 39, 41, 43, 45 de chaque détecteur, comme indiqué également sur la figuré 1. Ces axes XX', YY' sont en fait dans un plan perpendiculaire à l'axe central du système ; cet axe central, sur les figures 1 et 2, est perpendiculaire au plan de ces figures et est symbolisé par le point M.

Les références 34 et 36 désignent les connexions qui permettent de prélever un signal sur chaque électrode.

Seules sont représentées les connexions sortant de l'élément détecteur B, mais des connexions similaires sortent des éléments A, C voisins de B, également du côté extérieur 56 de la couronne 54.

On constate que ces connexions sont toutes situées du côté extérieur 56 de cette couronne de détecteurs, côté défini notamment par les arêtes 39, 45.

On constate dans cet exemple que chaque électrode, ou presque chaque électrode (on peut avoir des électrodes inclinées mais qui, pour certaines, ne sont réparties que sur un seul détecteur), court à la surface de au moins deux ou même de plus de deux détecteurs.

Sur les figures 7A et 7B sont représentées deux rangées de détecteurs (A, B, C pour la première rangée, D, E, F pour la deuxième). Sur cet exemple, chaque électrode est présente simultanément sur trois blocs détecteurs.

Si le dispositif ne comportait qu'une seule rangée (par exemple A, B, C), certaines électrodes seraient présentes sur deux détecteurs, les autres n'étant présentes que sur un détecteur malgré leur inclinaison.

Le nombre de blocs traversés dépend des rayons intérieurs et extérieurs de la couronne 54 de détecteurs du système, de la taille des blocs détecteurs et de l'inclinaison $\beta$ des électrodes.

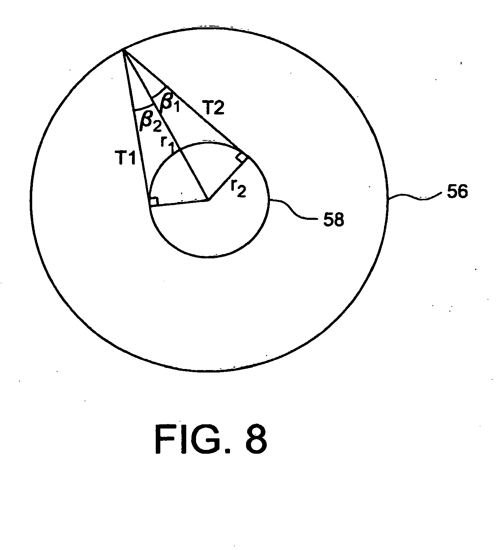

Ainsi, un système TEP pour petit animal possède un rayon intérieur r2 pouvant aller de 25mm à 75mm, le rayon extérieur r1 de la couronne dépendant de l'épaisseur (indiquée par L3 sur fig. 7A) du matériau détecteur (les rayons r1 et r2 sont mesurés comme indiqué sur la figure 1 ou là figure 8 par exemple). Pour un semi-conducteur tel que le CdZnTe, cette épaisseur est comprise entre 20mm et 100mm pour assurer un pouvoir d'arrêt des rayonnements de 65 % à 99 %, ce qui fournit un rayon extérieur de 45mm à 175mm.

Les valeurs de l'angle d'inclinaison $\beta$ des cathodes sont limitées par les deux tangentes (T1 et T2) à la circonférence intérieure 58 du système, comme illustré sur la figure 8.

Les électrodes d'une face sont inclinées, par rapport à un rayon du pourtour extérieur 56, d'un angle $\beta1$, $\beta2$ inférieur à celui délimité par ledit rayon et une tangents T1, T2 au cercle intérieur 58 qui rejoint ledit rayon au point d'intersection de ce rayon sur le cercle extérieur 56.

En effet; si l'on dépasse cette inclinaison, les électrodes d'une même face débouchent alors sur l'extérieur de là couronné d'une manière telle que les autres électrodes ne pourront avoir la même configuration. Un système avec $\beta1<\beta<90°$ est également possible.

Dans l'exemple ci-dessus, l'angle d'inclinaison maximum est obtenu pour un rayon intérieur r2 grand (75mm) et le rayon extérieur r1 associé le plus petit (95mm), soit :

$$\beta1 = \beta2 = \beta = \arcsin(75/95) \approx 52,5°$$

L'angle d'inclinaison des anodes est limité par les mêmes valeurs.

Au final, les deux familles d'électrodes peuvent être, respectivement, inclinées d'un angle $\beta$, $\beta'$ compris entre 0 et, respectivement, $\beta1$ et $\beta2$ (respectivement -52, 5° et 52, 5° dans l'exemple ci-dessus). $\beta$ et $\beta'$ ne soit pas nécessairement égaux.

Pour des raisons de tenue mécanique, les longueurs des côtés (L1 et/ou L2 (voir fig. 7A)) des blocs détecteurs sont de préférence comprises entre 5mm et 30mm pour le CdZnTe.

Le tableau I ci-dessous rassemble, pour divers matériaux, et diverses configuration géométriques, des valeurs de $\beta1$ et $\beta2$.

Tableau I

|  |  | L3(mm) | R1 (mm) | R2 (mm) | $\beta1=\beta2$ |
|---|---|---|---|---|---|
| **Si** | Min | 40 | 55 | 15 | 45,6° |
|  | Max | 270 | 370 | 100 |  |
| **Ge** | Min | 20 | 35 | 15 | 56,4° |
|  | Max | 130 | 230 | 100 |  |
| **GeAs** | Min | 20 | 35 | 15 | - 56,4° |
|  | Max | 130 | 230 | 100 |  |
| **CdTe: C1** | Min | 20 | 35 | 15 | 56,4° |
|  | Max | 100 | 200 | 100 |  |
| **CdZnTe** | Min | 20 | 35 | 15 | 56,4° |
|  | Max | 100 | 200 | 100 |  |
| **HgI$_2$** | Min | 10 | 25 | 15 | 65,4° |
|  | Max | 70 | 100 | 100 |  |

L1 serà fonction de L3 (voir figure 7A) et du nombre de couronnes superposées (par exemple : deux couronnes ABC et DEF sont superposées sur la figure 7A). En ce qui concerne L2, elle vaudra par exemple 270 mm dans le premier cas (Si), 100 mm dans le deuxième (Ge), 130 mm dans le troisième (130 mm), 30 mm dans le quatrième (CdTe : Cl) et le cinquième (CdZnTe) et 70 mm dans le dernier cas (HgI2).

Suivant les dimensions retenues et l'angle $\beta$, le nombre de blocs parcourus par un segment ou une électrode varie.

Pour une couronne d'épaisseur de détection L3 = 20mm, composée de blocs de 20mm (= L1) avec un angle d'inclinaison $\beta$ proche de 0°, certaines pistes ou électrodes seront présentes sur un seul détecteur.

En revanche, pour une couronne de 100mm d'épaisseur, composée de 20 blocs de 5mm, des électrodes peuvent être présentes sur 20 détecteurs.

Chaque électrode peut donc être présente sur 1 à 20 blocs détecteurs.

Cette disposition simultanée d'électrodes sur plusieurs détecteurs permet également de limiter le nombre total de voies de lecture électronique requises.

Sur l'exemple des figures 7A et 7B, Chaque électrode est commune à 3 blocs.

Cependant, les voies de traitement électroniques 34, 36 sont présentes uniquement sur les blocs A, B et C. Les blocs D, E et F ne sont en fait que des extensions de A, B et C (respectivement), afin d'assurer un pouvoir d'arrêt supérieur.

Par conséquent, chaque électrode est en fait commune à 2 blocs effectifs de détection, que l'on baptise AD (A+D), BE (B+E) et CF (C+F).

Sur la figure 7A, le bloc effectif BE est parcouru par 22 anodes (11 partant de l'extrémité inférieure 41 du bloc D, et 11 du bloc E) et 22 cathodes différentes (11 partant de l'extrémité inférieure 49 du bloc E, et 11 du bloc F).

Cependant, comme l'illustre la figure 7A, seulement 50 % des surfaces couvertes par les plans anodique et cathodique ainsi définis se croisent sur le bloc BE.

Le nombre de voxels subdivisant ce bloc est donc :

```
(22x22)/2 = 242 voxels
```

Avec seulement n voies électroniques, ici 22, par bloc détecteur effectif, on peut donc lire $n^2/2$ voxels, ici 242.

Un dessin classique d'électrodes se croisant orthogonalement permet de lire $N^2$ voxels avec 2N voies électroniques, soit 121 voxels avec 22 voies (11 cathodes et 11 anodes).

Dans l'exemple présenté, on multiplie donc par 2 le nombre de voxels observés pour un même nombre de voies électroniques par rapport au schéma classique tel que celui de la figure 6.

Dans le cas général où chaque électrode traverse I blocs effectifs de détection possédant N voies anodiques et N voies cathodiques, le nombre de voxels subdivisant un bloc est :

$$(I \times N) \times (I \times N)/I = I \times N^2 \text{ voxels}$$

Le gain est donc directement proportionnel au nombre d'électrodes mises en commun entre les blocs.

L'invention permet de mettre en place des moyens de connexion 34, 36 présents sur l'ensemble des extrémités des pistes, mais sur un seul côté, le côté extérieur, des détecteurs (du côté des arêtes 39 et 45 des blocs A et C de la figure 7A, ou encore du côté 56 de la couronne 54).

Cette connectique est simple de mise en oeuvre entre les électrodes et les chaînes ou les moyens de traitement électronique, et, en outre, elle ne réduit pas l'espace dédié à la détection.

L'invention permet en outre de densifier la segmentation des détecteurs, tout en limitant fortement le nombre de chaînes électroniques requises.

Dé plus, un module de détection peut être constitué de la superposition de plusieurs couronnes telles que celle décrite en figures 7A, 7B, superposées perpendiculairement à la figure 7A, 7B pour former Un cylindre. Chaque détecteur présente par exemple une distance interélectrode inférieure ou égale à 2 mm. Il peut être séparé des détecteurs d'une autre couronne, par exemple, de quelques dizaines de micromètres pour permettre l'isolation des électrodes.

Là réalisation d'électrodes réparties sur plusieurs détecteurs comporte les étapes suivantes.

On met d'abord en place des contacts métalliques sur les blocs de détection. Par un contrôle métrologique, on aligne ces contacts entre détecteurs, destinés à faire partie d'un même ensemble de détection. La technique de réalisation est ensuite la même que pour les détecteurs classiques.

On réalisé ensuite un masque spécifique couvrant plusieurs détecteurs, de manière similaire à ce qu'on réalise pour la fabrication de tous types de détecteur avec contact métallique, sauf pour l'angle d'inclinaison β. Les techniques demeurent sinon celles couramment employées.

Un dispositif selon l'invention peut être relié à des moyens usuels de traitement des signaux prélevés sur les électrodes, pour traiter les signaux de manière usuelle, le nombre de voies de mesure étant adapté au nombre de voxels à mesurer.

## Revendications

1. Dispositif détecteur de type tomographe, comportant une pluralité d'éléments détecteurs (A--F), disposés en forme de couronne, des électrodes (30, 32) sur une face avant et une face arrière formant anode et cathode de ces éléments, chaque électrode étant munie de moyens de connexion (34, 36), l'ensemble de ces moyens de connexion étant localisés sur le pourtour extérieur de ladite couronne d'éléments détecteurs, les électrodes ayant la forme de segments, l'ensemble des segments recouvrant une face d'un élément détecteur étant disposé selon une direction inclinée par rapport à l'ensemble des segments recouvrant l'autre face dudit élément détecteur.

2. Dispositif selon la revendication 1, la couronne ayant un pourtour intérieur (58) sensiblement en forme de cercle, un pourtour extérieur (56) sensiblement en forme de cercle, ces deux cercles étant concentriques, les segments étant inclinés, par rapport à un rayon du pourtour extérieur (56), d'un angle (β1, β2) inférieur à celui délimité par ledit rayon et une tangente (T1, T2) au pourtour intérieur (58) qui rejoint ledit rayon au point d'intersection de ce rayon sur le pourtour extérieur (56).

3. Dispositif détecteur de type tomographe, **caractérisé en ce qu'**il comporte :

   - une pluralité, d'éléments détecteurs (A-F), disposés en forme de couronne, cette couronne ayant un pourtour intérieur (58) en forme de cercle, un pourtour extérieur (56) en forme de cercle, ces deux cercles étant concentriques,

   - des électrodes (30, 32) sur une face avant et une face arrière formant anode et cathode de ces éléments détecteurs, les électrodes d'une face étant inclinées, par rapport à un rayon du pourtour extérieur (56), d'un angle (β1, β2) inférieur à celui délimité par ledit rayon et une tangente (T1, T2) au pourtour intérieur (58) qui rejoint ledit rayon au point d'intersection de ce rayon sur le pourtour extérieur (56).

**4.** Dispositif selon la revendication 3, chaque électrode étant munie de moyens de connexion (34, 36), l'ensemble de ces moyens de connexion étant localisés sur le pourtour extérieur de ladite couronne d'éléments détecteurs.

**5.** Dispositif selon l'une des revendications 1 à 4, au moins une partie des électrodes recouvrant au moins deux détecteurs.

**6.** Dispositif selon l'une des revendications 1 à 5, au moins une partie des électrodes recouvrant entre un et 20 détecteurs.

**7.** Dispositif selon l'une des revendications 1 à 6, les électrodes (30, 32) des faces avant et arrière des éléments détecteurs étant disposées en forme d'éventails, définissant deux éventails croisés.

**8.** Dispositif selon l'une des revendications 1 à 7, les éléments détecteurs étant en matériau semi-conducteur.

**9.** Dispositif selon la revendication 8, les éléments détecteurs étant en matériau CdZnTe, ou CdTe, ou GaAs, ou Ge, ou HgI2, ou Si.

**10.** Dispositif selon l'une des revendications 1 à 9, la couronne de détecteurs ayant un rayon intérieur compris entre 15 mm et 100 mm.

**11.** Dispositif selon l'une des revendications 1 à 10, la couronne de détecteurs ayant un rayon extérieur compris entre 35 mm et 400 mm.

**Claims**

**1.** Tomograph type detection device, comprising a plurality of detection elements (A-F), arranged in the form of a ring, electrodes (30, 32) on a front face and a back face of these elements, forming an anode and a cathode, each electrode being provided with connection means (34, 36), all these connection means being located on the external periphery of said ring of detection elements, the electrodes being in the form of segments, all segments covering a face of a detection element being arranged along a direction inclined from the assembly of segments covering the other face of said detection element.

**2.** Device according to claim 1, wherein the ring has an inside periphery (58) approximately in the form of a circle, an external periphery (56) approximately in the form of a circle, these two circles being concentric, the segments being inclined with respect to a radius of the external periphery (56), with an angle (β1, β2) less than the angle delimited by said radius and a tangent (T1, T2) to the internal periphery (58) that meets said radius at the intersection point of this radius on the outside periphery (56).

**3.** Tomograph type detection device, **characterized in that** it comprises:

- a plurality of detection elements (A-F), arranged in the form of a ring, this ring having an inside periphery (58) forming a circle, an outside periphery (56) forming a circle, these two circles being concentric,
- electrodes (30, 32) on a front face and a back face of these detection elements, forming an anode and a cathode, the electrodes on one face being inclined with respect to a radius of the outside periphery (56), with an angle (β1, β2) less than the angle delimited by said radius and a tangent (T1, T2) to the inside periphery (58) that meets said radius at the intersection point of this radius on the outside periphery (56).

**4.** Device according to claim 3, wherein each electrode is provided with connection means (34, 36), all of these connection means being located on the outside periphery of said ring of detection elements.

**5.** Device according to one of claims 1 to 4, wherein at least part of the electrodes covers at least two detectors.

**6.** Device according to one of claims 1 to 5, wherein at least part of the electrodes covers between one and 20 detectors.

**7.** Device according to one of claims 1 to 6, wherein the electrodes (30, 32) on the front face and the back face of the detector elements are arranged in the form of fans defining two crossed fans.

8. Device according to one of claims 1 to 7, wherein the detection elements are made of a semiconducting material.

9. Device according to claim 8, wherein the detection elements are made of CdZnTe, or CdTe, or GaAs, or Ge, or HgI2, or Si.

10. Device according to one of claims 1 to 9, wherein the inside radius of the ring of detectors is between 15 mm and 100 mm inclusive.

11. Device according to one of claims 1 to 10, wherein the outside radius of the ring of detectors is between 35 mm and 400 mm inclusive.

**Patentansprüche**

1. Detektionsvorrichtung des Typs Tomograph, umfassend eine Vielzahl in Form eines Rings angeordneter Detektorelemente (A-F), Elektroden (30, 32), Anode und Kathode auf einer Vorderseite und einer Rückseite dieser Detektorelemente bildend, wobei jede Elektrode Anschlusseinrichtungen (34, 36) umfasst, die Gesamtheit dieser Anschlusseinrichtungen am Außenumfang des Detektorelemente-Rings lokalisiert ist, die Elektroden die Form von Segmenten haben, und die Gesamtheit der Segmente, die eine Seite eines Detektorelements bedecken, gemäß einer Richtung angeordnet sind, die geneigt ist in Bezug auf die Gesamtheit der Elemente, welche die andere Seite des genannten Detektorelements bedecken.

2. Vorrichtung nach Anspruch 1, wobei der Ring einen im Wesentlichen kreisförmigen Innenumfang (58) und einen im Wesentlichen kreisförmigen Außenumfang (56) aufweist, diese beiden Kreise konzentrisch sind und die Segmente in Bezug auf einen Radius des Außenumfangs (56) um einen Winkel (β1, β2) geneigt sind, der kleiner ist als derjenige, den der genannte Radius und eine Tangente (T1, T2) zum Innenumfang (58) begrenzen, die den genannten Radius in dem Schnittpunkt zwischen diesem Radius und dem Außenumfang (56) erreicht.

3. Detektorvorrichtung des Typs Tomograph,
   **dadurch gekennzeichnet, dass** sie umfasst:

   - eine Vielzahl Detektorelemente (A-F), ringförmig angeordnet, wobei dieser Ring einen kreisförmigen Innenumfang (58) und einen kreisförmigen Außenumfang (56) aufweist und diese beiden Kreise konzentrisch sind,
   - Elektroden (30, 32), die Anode und die Kathode auf einer Vorderseite und einer Rückseite dieser Detektorelemente bildend, wobei die Elektroden einer Seite in Bezug auf einen Radius des Außenumfangs (56) um einen Winkel (β1, β2) geneigt sind, der kleiner ist als derjenige, den der genannte Radius und eine Tangente (T1, T2) zum Innenumfang (58) begrenzen, die den genannten Radius in dem Schnittpunkt zwischen diesem Radius und dem Außenumfang (56) erreicht.

4. Vorrichtung nach Anspruch 3, wobei jede Elektrode Anschlusseinrichtungen (34, 36) umfasst und die Gesamtheit dieser Anschlusseinrichtungen am Außenumfang des genannten Detektorelemente-Rings lokalisiert ist.

5. Vorrichtung nach einem der Ansprüche 1 bis 4, wobei wenigstens ein Teil der Elektroden wenigstens zwei Detektoren bedeckt.

6. Vorrichtung nach einem der Ansprüche 1 bis 5, wobei wenigstens ein Teil der Elektroden zwischen einem und 20 Detektoren bedeckt.

7. Vorrichtung nach einem der Ansprüche 1 bis 6, wobei die Elektroden (30, 32) der Vorder- und der Rückseite der Detektorelemente fächerförmig angeordnet sind, zwei gekreuzte Fächer definierend.

8. Vorrichtung nach einem der Ansprüche 1 bis 7, wobei die Detektorelemente aus Halbleitermaterial sind.

9. Vorrichtung nach Anspruch 8, wobei die Detektorelemente aus CdZnTe- oder CdTe- oder GaAs- oder Ge- oder Hgl2- oder Si-Material sind.

10. Vorrichtung nach einem der Ansprüche 1 bis 9, wobei der Detektorenring einen zwischen 15 mm und 100 mm enthaltenen Innenradius hat.

11. Vorrichtung nach einem der Ansprüche 1 bis 10, wobei der Detektorenring einen zwischen 35 mm und 400 mm enthaltenen Außenradius hat.

# FIG. 1

# FIG. 2

FIG. 3

FIG. 4

# FIG. 5

# FIG. 6

FIG. 7A

FIG. 7B

FIG. 8

## RÉFÉRENCES CITÉES DANS LA DESCRIPTION

**Documents brevets cités dans la description**

- EP 1408347 A1

**Littérature non-brevet citée dans la description**

- **W.H. WONG.** Designing a stratified detection system for PET caméras. *IEEE Transaction on Nuclear science,* 1986, vol. 33 (1), 591-596